# EUROPÄISCHE PATENTSCHRIFT

(11) **EP 0 965 355 B1**
(45) Veröffentlichungstag und Bekanntmachung des Hinweises auf die Patenterteilung: **04.05.2005**
(21) Anmeldenummer: 99101672.6
(22) Anmeldetag: 05.02.1999
(51) Int. Cl.: A61M 15/00, A61M 16/00, A62B 7/02

(54) **Gesteuerter Inhalator**
Controlled inhaler
Inhalateur contrôlé

(30) Priorität: 17.06.1998 DE 19826933; 06.11.1998 DE 19851279
(43) Veröffentlichungstag der Anmeldung: 22.12.1999
(73) Patentinhaber: GSF-Forschungszentrum für Umwelt und Gesundheit GmbH, 85764 Oberschleissheim (DE)
(72) Erfinder: Scheuch, Gerhard, Dr., 35285 Gemünden (DE); Sommerer, Knut, 81241 München (DE)
(74) Vertreter: Reinhard - Skuhra - Weise & Partner GbR

(56) Entgegenhaltungen:
- EP-A- 0 143 208
- GB-A- 949 074
- GB-A- 2 033 967
- US-A- 4 821 712
- US-A- 5 056 511
- US-A- 5 427 091

## Beschreibung

Die Erfindung betrifft eine Vorrichtung zum gesteuerten inhalatorischen Einbringen von dosierten Medikamenten-Aerosolen in die Lunge.

Die Applikation von Medikamenten in Aerosolform durch Inhalation in die Lunge des Menschen gewinnt zunehmend an Bedeutung. Dabei besteht das Problem, daß eine schnelle Inhalation die Abscheidung der Teilchen am Kehlkopf und im Mund fördert und das Eindringen der Aerosolteilchen in die Lunge verhindert. Ungünstig ist es weiterhin, wenn das zu applizierende Aerosol nicht quantifiziert ist, weil gerade die möglichst kontrollierbare und insbesondere langsame Inhalation die Deposition der Aerosolteilchen in der Lunge fördert. Wenn der Patient mit beliebiger Geschwindigkeit atmen kann, kommt es in nachteiliger Weise dazu, daß die Deponierung der Partikel in der Lunge immer eine große Variation aufweist. Bei unkontrollierter Inhalation mit bisher bekannten Vorratskammern (Spacern) wird vom Patienten sowohl der Fluß (also die Atemgeschwindigkeit) als auch das Atemzugvolumen variiert.

In der GB-A-2 033 967 ist eine Pumpeinrichtung zur Luftversorgung eines Patenten offenbart, die eine flexible Außenmembran und eine flexible Innenmembran mit Einlaß- und Auslaßventil aufweist, wobei das Auslaßventil mit einem Mundstück versehen ist und eine Betätigung der Pumpeinrichtung durch pulsierende Druckgaszufuhr in den Raum zwischen beiden Membranen vorgenommen wird.

In der US-A-4 821 712 ist ein tragbares Notatmungsgerät offenbart, das einen äußeren Behälter aufweist, in dem eine Blase angeordnet ist, die mit einem Mundstück verbunden ist und durch Sauerstoffgas unter Druck aufblähbar ist. Die Druckzufuhr zu dem Beutel ist durch eine manuell betätigbare Anordnung aus Auslaßventil und Einlaßventil vorgesehen, wobei für nicht benötigte Atemluft eine Einleitung in den Raum zwischen Behälter und Blase vorgesehen ist.

In der US-A-5 427 091 ist ein Atmungsgerät ähnlich dem aus der GB-2 033 967 bekannt, bei dem in einem flexiblen Behälter eine Blase angeordnet ist, die mit Sauerstoff befüllbar ist und die durch externes Einblasen in den Raum zwischen Behälter und Blase zur Abgabe von Sauerstoff unter Druck in ein Mundstück komprimierbar ist.

Nachteilig ist bei allen vorgenannten Druckschriften, daß mit diesen kein kontrolliertes Inhalieren zur Applikation von Medikamenten in Aerosolform möglich ist.

Der Erfindung liegt demgemäß die Aufgabe zugrunde, eine Vorrichtung der eingangs genannten Gattung derart weiterzubilden, daß bei einfacher Handhabung ein problemloses kontrolliertes Inhalieren ermöglicht wird.

Erfindungsgemäß wird diese Aufgabe durch die im Anspruch 1 genannten Merkmale gelöst. Bevorzugte Merkmale, die die Erfindung vorteilhaft weiterbilden, sind den nachgeordneten Patentansprüchen zu entnehmen.

Durch die erfindungsgemäßen Maßnahmen wird vorteilhaft eine Atemflußlimitierung ermöglicht, durch die die Einstellung eines maximalen Inhalationsflusses des Probanten ermöglicht und dadurch die Lungendeposition von Aerosolteilchen erhöht wird. Dabei sind durch verschiedene Fluß-Limitierungen (wie kritische Düse, einstellbare Düse) unterschiedliche Flußeinstellungen möglich, wodurch auch eine Anpassung an die eingesetzten Medikamente und die Möglichkeiten des Patienten erfolgen kann.

Zur Begrenzung des inhalierbaren Volumens ist ein Behältnis vorzugsweise in Form eines Ballons oder Beutels vorgesehen, wobei durch die vorgegebene Anfangsfüllung des Behältnisses eine quantifizierbare Aerosolmenge einstellbar ist.

Vorteilhaft ist weiterhin, daß die Koordinierung des Patienten durch die erfindungsgemäße Vorrichtung insoweit begrenzt wird, als zunächst eine bestimmte Füllung vorgegeben und dann in aller Ruhe entleert wird. Dabei kann durch einen vorzugsweise durchsichtigen Aufbau des Behältnisses bzw. Ballons der Patient vorteilhaft auch optisch kontrollieren, ob er wirklich das vorgegebene Volumen geleert hat und auch einzelne Atemzüge am Schrumpfen des Ballons nachverfolgen.

Vorteilhaft sind verschiedene Volumina für einzelne Anwendungsgebiete vorwählbar, beispielsweise 50-150 ml für bevorzugte Deponierung in den konduktiven Atemwegen der Lunge, oder 500-5000 ml für bevorzugte maximale Deponierung des Aerosols im Alveolarbereich der Lunge.

Der Aufbau der Vorrichtung ist außerordentlich einfach verifizierbar und tritt dem Patienten in einer Form gegenüber, die ohne verunsichernde Elektronik erscheint und damit auch die Akzeptanz selbsterklärend (sichtbare Entleerung eines Ballons) fördert.

Bei der erfindungsgemäßen Vorrichtung wird das als Beutel oder Ballon ausgebildete Behältnis mit einer bestimmten Menge Aerosol gefüllt, wobei die Menge durch die Größe des Behältnisses vorbestimmt ist. Das Aerosol kann in herkömmlicher Weise über Dosieraerosole, Trockenpulverinhalatoren, Ultraschall- oder Verneblersysteme erzeugt werden. Dabei befindet sich gemäß einer bevorzugten Ausgestaltung der Erfindung ein Ballon in einem durchsichtigen Behälter, beispielsweise aus Plexiglas, welches in seiner Formgestaltung eine bequeme Handhabung durch den Patienten ermöglicht, beispielsweise durch eine Handhabe in Form einer Griffmulde.

Der Patient inhaliert dann den Inhalt des Beutels, wobei er durch ein einstellbares Ventil bzw. eine kritische Düse gezwungen wird, den Beutel langsam zu entleeren. Dieser Inhalationsfluß ist durch Einstellung einer kritischen Düse oder eines verstellbaren Ventils veränderbar, wobei der Patient eine direkte optische Kontrolle über die Menge des eingeatmeten Aerosols insoweit hat, als der Beutel nach Einatmung einer quantivizierten vorbestimmten Menge des Aerosols leer ist.

Für das Gehäuse sind je nach Anwendung verschiedene volumetrische Größen vorgesehen. Wenn beispielsweise das Aerosol ausschließlich oder bevorzugt in den konduktiven Atemwegen abgelagert werden soll, bieten sich kleine Gehäuse mit Volumina von 50 - 250 ml an, während bei Forderung nach einer Deponierung hauptsächlich oder ausschließlich im Alveolarbereichgehäuse von 600 - 5000 ml vorgesehen sein können. Dabei gilt die Regel, daß je tiefer die Inhalation ist, desto effektiver und umso mehr Aerosol gelangt in den Alveolarbereich.

Gemäß den beanspruchten Merkmalen ist vorteilhaft somit der Behälter in einem Gehäuse angeordnet, wobei das Gehäuse zur Kontrolle des Füllstandes des Behältnisses wenigstens teilweise durchsichtig ist, wobei das Behältnis mit einem Inhalationsmundstück verbunden ist und das Gehäuse ein Befüllventil aufweist. Die Einstelleinrichtung für den Inhalationsfluß ist mit einem Verstellventil oder einer sogenannten kritischen Düse gebildet.

Das Behältnis ist wenigstens teilweise elastisch, bevorzugt auch mit Rückverformungselastizität, und vorzugsweise als Beutel oder Ballon ausgebildet, und das Gehäuse weist eine Handhabe auf, die vorzugsweise in die Form eines Handgriffs integriert ist. Diese Handhabe kann vorzugsweise als Griffmulde ausgebildet sein, und das Befüllventil ist vorteilhaft im Bereich dieser Handhabe angeordnet.

Nachfolgend wird die Erfindung anhand eines schematisch dargestellten Ausführungsbeispiels unter Bezugnahme auf die beigelegte Figur näher erläutert.

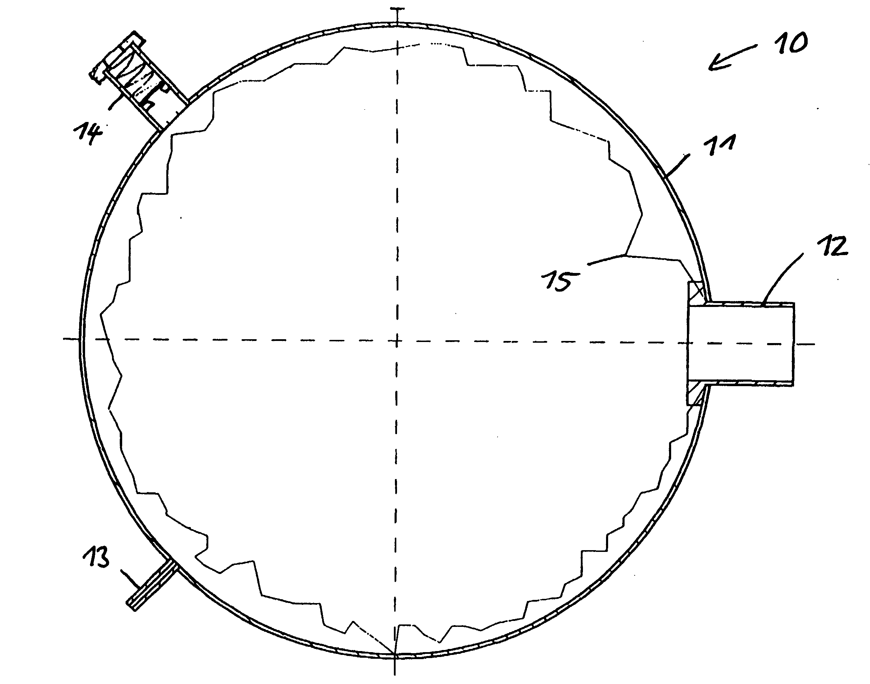

Die beigefügte Figur zeigt schematisiert im Querschnitt eine Ausgestaltung einer erfindungsgemäßen Vorrichtung 10. Die Vorrichtung 10 besteht aus einem kugelförmigen Gehäuse 11, das aus einem durchsichtigen Material, wie beispielsweise Plexiglas hergestellt ist. Das Gehäuse 11 weist weiterhin ein vorstehendes Mundstück 12, eine als dünner Rohrstutzen mit kleinem Querschnitt ausgebildete kritische Düse 13 zur Flußlimitierung sowie ein Ventil 14 auf, das als Einwegventil ausgebildet ist und aus dem Inneren des Behälters 11 nach außen öffnet. Im Behälter selbst ist ein Behältnis in Form eines Ballons 15 schematisiert angedeutet, der geschlossen ist und mit dem Mundstück 12 verbunden ist.

Zur Benutzung der Vorrichtung wird zunächst ein zu verabreichendes Aerosolmedikamentenvolumen festgelegt und dann der Ballon 15 über das Mundstück 12 entsprechend befüllt, wobei das Ventil 14 ein Entfalten des Ballons 15 im Inneren des durchsichtigen Behälters 11 ermöglicht.

Für die Vornahme der gewünschten Inhalation ergreift nun der Patient das Gehäuse an einer nicht dargestellten Handhabe, atmet aus und nimmt das Mundstück 12 (nach Entfernen einer abdichtenden Folie) in den Mund. Für das Einatmen wird dann die kritische Düse 13 zur kontrollierten Aerosolausgabe aus dem Ballon 15 freigegeben. Anstelle der kritischen Düse kann auch ein einstellbares Ventil vorgesehen sein, wobei durch den Strömungsquerschnitt der kritischen Düse der Außenluftzutritt zum Inneren des Behälters 11 und damit auch die mögliche Kontraktion des Ballons 15 bestimmt, wodurch eine einfache Kontrolle des Atemzugvolumens ermöglicht wird. Gleichzeitig ist über die Füllung des Ballons selbst ein zu applizierendes Medikamentenaerosolvolumen vorgegeben, wobei bei Verwendung eines einstellbaren Ventils statt der kritischen Düse 13 auch die Tiefe des jeweiligen Atemzugs und damit der gewünschte Applikationsbereich für das Medikamentenaerosol vorgegeben werden kann.

Zu einer bevorzugten Ausführungsform der Vorrichtung zum gesteuerten inhalatorischen Einbringen von dosierten Medikamenten in die Lunge ist das Behältnis 15 derart elastisch ausgebildet, daß es sich nach der Entnahme des Aerosols selbstätig in seine ursprüngliche Form vor der Entnahme zurückformt. Dadurch ist es möglich, das entleerte Behältnis 15 von außen über das Inhalationsmundstück 12 mit Medikamenten/Aerosolen in einfacher Weise erneut zu füllen.

Eine nicht dargestellte Verschlußfolie ist auf das Mundstück 12 zur Abdichtung und zum hygienischem Schutz des gefüllten Behältnisses 15 aufgebracht und wird vor Inbenutzungsnahme abgezogen.

Die Vorrichtung läßt sich außerordentlich einfach mit einem Plexiglasgehäuse und einem Gummiballon in einer Preiskategorie herstellen, die in den Bereich von Einwegartikel reicht.

## Patentansprüche

1. Vorrichtung zum gesteuerten inhalatorischen Einbringen von dosierten Medikamenten in die Lunge, bestehend aus
einem Gehäuse (11);
einem geschlossenen Behältnis (15), das in dem Gehäuse (11) angeordnet und mit einem vorbestimmbaren Aeorosolvolumen befüllbar ist; und aus
einer Steuereinrichtung (13), durch die eine Umgebungsluftströmung in das Gehäuse (11) zwischen diesem und dem Behältnis (15) zur Steuerung der Kontraktion des Behältnisses (15) und damit einer Inhalationsströmung aus dem Behältnis (15) begrenzbar ist.
**dadurch gekennzeichnet,**
**daß** die Steuereinrichtung (13) als Verstellventil oder als kritische Düse ausgebildet ist.

2. Vorrichtung nach Anspruch 1, **dadurch gekennzeichnet, daß** das Behältnis (15) mit einem Inhalationsmundstück (12) verbunden ist.

3. Vorrichtung nach einem der vorangehenden Ansprüche, **dadurch gekennzeichnet, daß** das Gehäuse (11) zur Kontrolle des Füllstandes des Behältnisses (15) wenigstens teilweise durchsichtig ist.

4. Vorrichtung nach einem der vorangehenden Ansprüche, **dadurch gekennzeichnet, daß** das Behältnis (15) wenigstens teilweise elastisch ist.

5. Vorrichtung nach einem der vorangehenden Ansprüche, **dadurch gekennzeichnet, daß** das Behältnis (15) aus einem sich nach Aeorosolentnahme elastisch in die Behältnisform rückverformenden Material besteht.

6. Vorrichtung nach Anspruch 4 oder 5, **dadurch gekennzeichnet, daß** das Behältnis (15) aus einem Beutel bzw. einem Ballon besteht.

7. Vorrichtung nach einem der vorangehenden Ansprüche, **dadurch gekennzeichnet, daß** das Gehäuse (11) ein Befüllventil (14) aufweist und daß die Steuereinrichtung (13) am Gehäuse (11) vorgesehen ist.

8. Vorrichtung nach Anspruch 7, **dadurch gekennzeichnet, daß** das Befüllventil (14) im Bereich einer an dem Gehäuse (11) vorgesehenen Handhabe angeordnet ist.

9. Vorrichtung nach einem der vorangehenden Anspruche, **dadurch gekennzeichnet, daß** das Gehäuse (11) in einem Handgriff integriert ist.

10. Vorrichtung nach einem der vorangehenden Ansprüche, **dadurch gekennzeichnet, daß** die Handhabe als Griffmulde ausgebildet ist.

## Claims

1. A device for a controlled inhalational administration of controlled-dosage drugs into the lungs, comprising
a housing (11);
a closed recipient (15), which is arranged within the housing (11) and is adapted to be charged with a predeterminable aerosol volume; and
a control means (13), adapted to limit an outside air flow in the housing (11) between the housing and the recipient (15) for controlling the contraction of the recipient (15) and thereby an inhalation flow out of the recipient (15), **characterised in that** the control means (13) is formed as an adjusting valve or a critical nozzle.

2. The device according to claim 1,
**characterised in that** the recipient (15) is connected to an inhalation mouth piece (12).

3. The device according to claim 1 or 2,
**characterised in that** the housing (11) is at least partly transparent for control of the charging level in the recipient (15).

4. The device according to one of the preceding claims,
**characterised in that** the recipient (15) is at least partly elastic.

5. The device according to one of the preceding claims,
**characterised in that** the recipient (15) consists of a material elastically resuming the original shape of the recipient after withdrawal of aerosol.

6. The device according to claim 4 or 5, **characterised in that** the recipient (15) consists of a bag or a balloon.

7. The device according to one of the preceding claims,
**characterised in that** the housing comprises a charging valve (14) and that said control means (13) is provided at the housing (11).

8. The device according to claim 7, **characterised in that** the charging valve (14) is arranged in the area of a handle provided at said housing (11).

9. The device according to one of the preceding claims,
**characterised in that** the housing (11) is integrated in a handle grip.

10. The device according to one of the preceding claims,
**characterised in that** the handle is designed as a recessed grip.

## Revendications

1. Dispositif d'introduction commandé par inhalation de médicaments dosés dans le poumon constitué
d'une enveloppe (11) ;
d'un récipient (15) fermé qui est disposé dans l'enveloppe (11) et qui peut être empli d'un volume d'aérosol pouvant être déterminé à l'avance ; et
d'un dispositif (13) de commande par lequel un courant d'air ambiant dans l'enveloppe (11), entre celle-ci et le récipient (15), peut être limité pour la commande de la contraction du récipient (15) et ainsi d'un courant d'inhalation sortant du récipient (15),
**caractérisé**
**en ce que** le dispositif (13) de commande est constitué en vannes de réglage ou en buses critiques.

2. Dispositif selon la revendication 1, **caractérisé en ce que** le récipient (15) communique avec un embout (12) d'inhalation.

3. Dispositif suivant l'une des revendications précédentes, **caractérisé en ce que** l'enveloppe (11) est transparente au moins en partie pour le contrôle du niveau de remplissage du récipient (15).

4. Dispositif suivant l'une des revendications précédentes, **caractérisé en ce que** le récipient (15) est élastique au moins en partie.

5. Dispositif suivant l'une des revendications précédentes, **caractérisé en ce que** le récipient (15) est en un matériau se redéformant élastiquement à la forme du récipient après prélèvement d'aérosol.

6. Dispositif suivant la revendication 4 ou 5, **caractérisé en ce que** le récipient (15) est constitué d'un sac ou d'un ballon.

7. Dispositif suivant l'une des revendications précédentes, **caractérisé en ce que** l'enveloppe (11) a une soupape (14) de remplissage et **en ce que** le dispositif (13) de commande est prévu pour l'enveloppe (11).

8. Dispositif suivant la revendication 7, **caractérisé en ce que** la soupape (14) de remplissage est disposée dans la zone d'une poignée prévue sur l'enveloppe (11).

9. Dispositif suivant l'une des revendications précédentes, **caractérisé en ce que** l'enveloppe (11) est intégrée dans une poignée.

10. Dispositif suivant l'une des revendications précédentes, **caractérisé en ce que** la poignée est constituée en une poignée encastrée.
